# EUROPÄISCHE PATENTSCHRIFT

(11) **EP 1 140 926 B1**
(45) Veröffentlichungstag und Bekanntmachung des Hinweises auf die Patenterteilung: **28.08.2002**
(21) Anmeldenummer: 00904871.1
(22) Anmeldetag: 04.01.2000
(51) Int. Cl.: C07D 413/12, C07D 413/14, A01N 43/82, A01N 43/88, C07D 231/20, C07D 249/12

(54) **AZADIOXACYCLOALKENE UND IHRE VERWENDUNG ZUR BEKÄMPFUNG VON SCHADPILZEN UND TIERISCHEN SCHÄDLINGEN**
AZADIOXACYCLOALKENES AND THEIR USE FOR COMBATING HARMFUL FUNGI AND ANIMAL PESTS
AZADIOXACYCLOALKENES ET LEUR UTILISATION POUR LUTTER CONTRE DES CHAMPIGNONS NUISIBLES ET DES PARASITES ANIMAUX

(30) Priorität: 13.01.1999 DE 19900892
(43) Veröffentlichungstag der Anmeldung: 10.10.2001
(73) Patentinhaber: BASF AKTIENGESELLSCHAFT, 67056 Ludwigshafen (DE)
(72) Erfinder: GEWEHR, Markus, D-56288 Kastellaun (DE); SAUTER, Hubert, D-68167 Mannheim (DE); MÜLLER, Bernd, D-67227 Frankenthal (DE); GRAMMENOS, Wassilios, D-67063 Ludwigshafen (DE); GYPSER, Andreas, D-68159 Mannheim (DE); PTOCK, Arne, D-67065 Ludwigshafen (DE); CULLMANN, Oliver, D-68199 Mannheim (DE); TORMO I BLASCO, Jordi, D-67117 Limburgerhof (DE); AMMERMANN, Eberhard, D-64646 Heppenheim (DE); STRATHMANN, Siegfried, D-67117 Limburgerhof (DE); LORENZ, Gisela, D-67434 Neustadt (DE); HARRIES, Volker, D-67227 Frankenthal (DE); GÖTZ, Roland, D-68809 Neulussheim (DE); GROTE, Thomas, D-67157 Wachenheim (DE)
(74) Vertreter: Fitzner, Uwe, Dr.
(86) Internationale Anmeldenummer: EP0000013
(87) Internationale Veröffentlichungsnummer: WO00042039

(56) Entgegenhaltungen:
- WO-A-94/19331
- WO-A-95/04728

## Beschreibung

Die vorliegende Erfindung betrifft Azadioxacycloalkene der Formel I, 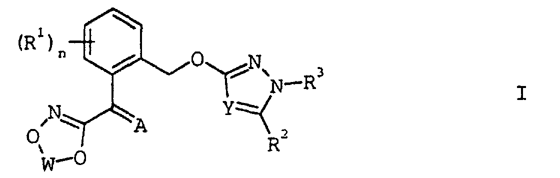 in der die Substituenten und der Index die folgende Bedeutung haben:
- Y: N oder CR^{a}, wobei
R^{a} für Wasserstoff, Halogen oder C₁-C₄-Alkyl steht;
- n: 0, 1, 2, 3 oder 4, wobei die Substituenten R¹ verschieden sein können, wenn n größer als 1 ist;
- R¹: Nitro, Cyano, Halogen, C₁-C₆-Alkyl oder
für den Fall, daß n größer 1 ist, zusätzlich eine an zwei benachbarte Ringatome gebundene Brücke, welche drei bis vier Glieder ausgewählt aus der Gruppe: 3 oder 4 Kohlenstoffatome, 2 bis 3 Kohlenstoffatome und 1 oder 2 Stickstoff-, Sauerstoff- und/oder Schwefelatome enthält, wobei diese Brücke gemeinsam mit dem Ring an den sie gebunden ist einen partiell ungesättigten oder aromatischen Rest bilden kann und weiterhin die Kohlenstoffatome der Brücke durch Halogenatome oder Methylgruppen teilweise oder vollständig substituiert sein können;
- R²: Wasserstoff, Nitro, Cyano, Halogen, C₁-C₄-Alkyl, C₁-C₄-Halogenalkyl, C₁-C₄-Alkoxy, C₁-C₄-Alkylthio oder C₁-C₄-Alkoxycarbonyl;
- R³: C₁-C₆-Alkyl, das unsubstituiert oder partiell oder vollständig halogeniert ist oder eine bis drei Gruppen R^{b} tragen kann,
R^{b} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino,
Di-C₁-C₆-alkylamino, C₂-C₆-Alkenyl, C₂-C₆-Alkenyloxy, C₃-C₆-Alkinyloxy und C₁-C₄-Alkylendioxy, welches halogeniert sein kann, C₃-C₆-Cycloalkyl, Phenyl, Naphthyl, 5-gliedriges Heteroaryl, enthaltend ein bis vier Stickstoffatome oder ein bis drei Stickstoffatome und ein Schwefel- oder Sauerstoffatom oder 6-gleidriges Heteroaryl, enthaltend ein bis vier Stickstoffatome,
wobei die cyclischen Systeme partiell oder vollständig halogeniert sein oder eine bis drei Gruppen R^{c} tragen können:
R^{c} Halogen, Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyl- oder Alkinylgruppen in diesen Resten 2 bis 8 Kohlenstoffatome enthalten;
C(=NOR^{d})-Γ₁-R^{d'}, wobei R^{d} für Wasserstoff oder C₁-C₆-Alkyl, Γ für Sauerstoff, Schwefel oder NR^{d} steht und 1 gleich 0 oder 1 ist;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Phenyl, Phenoxy, Phenylthio, Phenyl-C₁-C₆-alkoxy, Phenyl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch eine bis drei Gruppen R^{b} substituiert sein können;
- A: =N-OR⁴, =CH-OR⁴ =CH-SR⁴ oder =CH-R⁵, wobei
R⁴ C₁-C₄-Alkyl oder C₁-C₄-Halogenalkyl,
R⁵ Halogen oder eine Gruppe R⁴ bedeutet und
- W: C₁-C₃-Alkylen, das unsubstituiert oder durch eine oder zwei Gruppen R^{e} substituiert ist, wobei
R^{e} für Halogen, Cyano, C₁-C₄-Alkyl, C₁-C₄-Halogenalkyl, C₂-C₄-Alkenyl, C₂-C₄-Halogenalkenyl, C₂-C₄-Alkinyl oder C₂-C₄-Halogenalkinyl, C₁-C₄-Alkoxy, C₁-C₄-Halogenalkoxy, C₂-C₄-Alkenoxy, C₂-C₄-Halogen-alkenoxy, C₂-C₄-Alkinoxy oder C₂-C₄-Halogenalkinoxy, C₁-C₄-Alkylcarbonyloxy steht.

Außerdem betrifft die Erfindung Verfahren zur Herstellung dieser Verbindungen, sie enthaltende Mittel sowie ihre Verwendung zur Bekämpfung von tierischen Schädlingen oder Schadpilzen.

Aus EP-A 378 755, EP-A 757 042, WO-A 94/19331, WO-A 95/29896 und WO-A 96/37480 sind 2-[Pyrazolyl-4-oxymethylen]- und 2-[Triazolyl-4-oxymethylen]-phenylessigsäureester und entsprechende Amide zur Bekämpfung von tierischen Schädlingen und Schadpilzen bekannt.

3-Phenylmethylen-azadioxacycloalkene mit orthoständigem Substituenten an dem Phenylrest werden in WO-A 95/04728, WO-A 97/27189, WO-A 98/17653, WO-A 98/25465, WO-A 98/40351, WO-A 98/40365, WO-A 98/45289, EP-A 846 691 und WO-A 97/00866 offenbart.

Der vorliegenden Erfindung lagen Verbindungen mit verbesserter Wirkung als Aufgabe zugrunde.

Demgemäß wurden die eingangs definierten Verbindungen gefunden. Desweiteren wurden Verfahren zu ihrer Herstellung, sie enthaltende Mischungen sowie Verfahren zur Bekämpfung von tierischen Schädlingen und Schadpilzen unter Verwendung der Verbindungen I gefunden.

Die Verbindungen der Formel I unterscheiden sich von den aus den oben genannten Schriften WO-A 95/04728, WO-A 97/27189, WO-A 98/17653, WO-A 98/25465, WO-A 98/40351, WO-A 98/40365, WO-A 98/45289, EP-A 846 691 und WO-A 97/00866 in der Methylenoxy-Pyrazolyl-, bzw. -Triazolylgruppierung am Phenylring und von den aus EP-A 378 755, EP-A 757 042, WO-A 94/19331, WO-A 95/29896 und WO-A 96/37480 bekannten Verbindungen in der Azadioxacycloalken-Gruppe. Die Verbindungen der Formel I weisen eine gegenüber den bekannten Verbindungen erhöhte Wirksamkeit gegen Schadpilze und tierische Schädlinge auf.

Die Verbindungen der Formel I können an sich analog der in WO-A 95/04728, WO-A 97/46542 und WO-A 98/17653 bzw. EP-A 378 755, EP-A 757 042, WO-A 94/19331 und WO-A 95/29896 beschriebenen Methoden erhalten werden.

Die Verbindungen I können auf verschiedenen Wegen erhalten werden, wobei es für die Synthese unerheblich ist, ob zunächst die Gruppe X, wobei # die Verknüpfung mit dem Phenylring kennzeichnet, 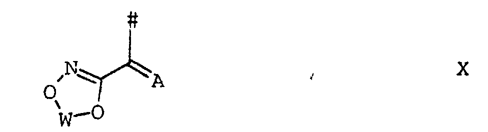 oder die Pyrazolyl-, bzw. Triazolylgruppierung aufgebaut wird.

Beispielsweise erhält man die Verbindungen der Formel I bevorzugt durch Umsetzung von Benzylverbindungen der Formel II mit Hydroxypyrazolen oder den entsprechenden Triazolen der Formel III. In Formeln II und III haben R¹, n, A, W, Y, R² und R³ die für die Formel I definierten Bedeutungen, in Formel II steht L für eine nucleophil austauschbare Gruppe, beispielsweise Halogen (z.B. Chlor oder Brom) oder ein Alkyl- oder Arylsulfonat (z.B. Methylsulfonat, Trifluormethylsulfonat, Phenylsulfonat und 4-Methylphenylsulfonat). 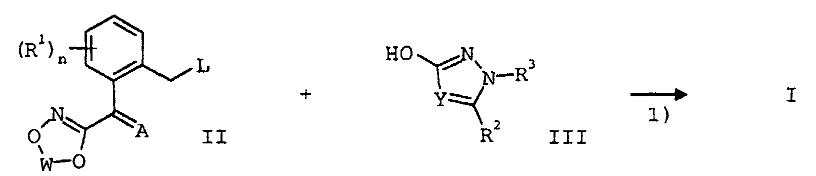
1) Die Veretherung der Verbindungen II und III wird üblicherweise in einem inerten organischen Lösungsmittel bei Temperaturen von 0°C bis 80°C, vorzugsweise bei 20°C bis 60°C, gegebenenfalls in Gegenwart einer Base durchgeführt.
   Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan und Tetrahydrofuran, Nitrile, Alkohole, Ketone wie Aceton und Methylethylketon sowie Dimethylsulfoxid, Dimethylformamid, Dimethylacetamid, 1,3-Dimethylimidazolidin-2-on und 1,2-Dimethyltetrahydro-2(1H)-pyrimidin, vorzugsweise Methylenchlorid, Aceton, Toluol, Methyl-tert.-butylether und Dimethylformamid. Es können auch Gemische der genannten Lösungsmittel verwendet werden.
   Als Basen kommen allgemein anorganische Verbindungen wie Alkalimetall- und Erdalkalimetallhydroxide (z.B. Lithiumhydroxid, Natriumhydroxid, Kaliumhydroxid und Calziumhydroxid), Alkalimetall- und Erdalkalimetalloxide, Alkalimetall- und Erdalkalimetallhydride, Alkalimetallamide, Alkalimetall- und Erdalkalimetallcarbonate (z.B. Lithiumcarbonat und Calziumcarbonat) sowie Alkalimetallhydrogencarbonate (z.B. Natriumhydrogencarbonat), metallorganische Verbindungen, insbesondere Alkalimetallalkyle , Alkylmagnesiumhalogenide sowie Alkalimetall- und Erdalkalimetallalkoholate (z.B. Natriummethanolat, Natriumethanolat, Kaliumethanolat, Kalium-tert.-Butanolat und Dimethoxymagnesium), außerdem organische Basen, z.B. tertiäre Amine wie Trimethylamin, Triethylamin, Tri-isopropylethylamin und N-Methylpiperidin, Pyridin, substituierte Pyridine sowie bicyclische Amine in Betracht. Besonders bevorzugt werden Natriumhydroxid, Kaliumcarbonat und Kalium-tert.-butanolat. Die Basen werden im allgemeinen äquimolar, im Überschuß oder gegebenenfalls als Lösungsmittel verwendet.
   Es kann für die Umsetzung vorteilhaft sein, eine katalytische Menge eines Kronenethers (z.B. 18-Krone-6 oder 15-Krone-5) zuzusetzen.
   Die Umsetzung kann auch in Zweiphasensystemen bestehend aus einer Lösung von Alkali- oder Erdalkalihydroxiden oder -carbonaten in Wasser und einer organischen Phase (z.B. aromatische und/oder halogenierte Kohlenwasserstoffe) durchgeführt werden. Als Phasentransferkatalysatoren kommen hierbei beispielsweise Ammoniumhalogenide und -tetrafluoroborate (z.B. Benzyltriethylammoniumchlorid, Benzyltributylammoniumbromid, Tetrabutylammoniumchlorid, Hexadecyltrimethylammoniumbromid oder Terabutylammoniumtetrafluoroborat) sowie Phosphoniumhalogenide (z.B. Tetrabutylphosphoniumchlorid und Tetraphenylphosphoniumbromid) in Betracht.
   Es kann für die Umsetzung vorteilhaft sein, zunächst das 3-Hydroxypyrazol oder -triazol mit der Base in das entsprechende Hydroxylat umzusetzen, welches dann mit dem Benzylderivat umgesetzt wird.
   Die Verwendung der Benzylverbindungen II, in denen L für Halogen steht, ist bevorzugt.
   Die Edukte werden im allgemeinen in äquimolaren Mengen miteinander umgesetzt. Es kann für die Ausbeute vorteilhaft sein, Hydroxypyrazole, bzw. -triazole in einem Überschuß bezogen auf II einzusetzen.
   Die für die Herstellung der Verbindungen I benötigten Benzylverbindungen II sind aus der Literatur bekannt [vgl. WO-A 95/04728; WO-A 98/17653]. Sie können beispielsweise auf dem folgenden Syntheseweg erhalten werden: 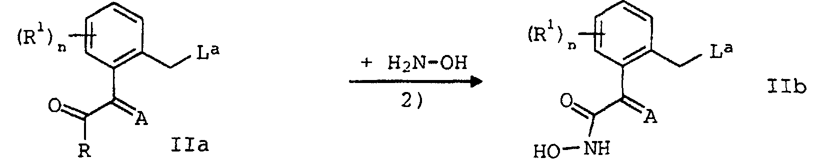 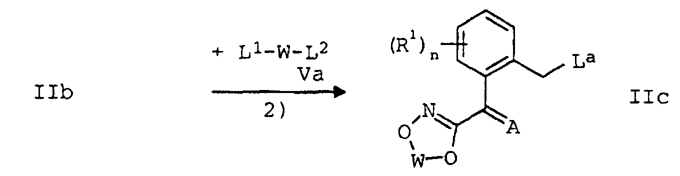
2) Benzylverbindungen der Formel IIa werden mit Hydroxylamin oder dessen Säureadditionssalz gegebenenfalls in Gegenwart einer Base oder eines wasserentziehenden Mittels, wie N,N'-Dicyclohexylcarbodiimid, oder in Gegenwart eines Kupplungsreagenzes, wie beispielsweise 2-Ethoxy-1-ethoxycarbonyl-1,2-dihydrochinolin (EEDQ), 1-Isobutyloxycarbonyl 2-isobutyloxy-1,2-dihydrochinolin (IIDQ) oder Benzotriazol-1-yloxy-tris-(dimethylamino)-phosphonium-hexafluorphoshat (BOP), zu den Hydroxamsäuren der Formel IIb umgesetzt, die sich durch Umsetzung mit Verbindungen der Formel IVa zu IIc cyclisieren lassen [vgl. WO-A 95/04728].
   In Formel IIa steht L^{a} für ein durch eine Schutzgruppe geschütztes Sauerstoffatom, R für Halogen, Alkylcarbonyloxy, OH, C₁-C₄-Alkoxy oder einen Aktivester wie gegebenenfalls substituiertes Phenoxy (wie beispielsweise p-Nitrophenoxy oder Pentafluorphenoxy), Succinimidoxy oder Isoharnstoff. In Formel Va hat W die in Anspruch 1 genannte Bedeutung und L¹, L² stehen jeweils für eine nucleophil austauschbare Gruppe wie Halogenid, C₁-C₄-Alkylsulfonat, C₁-C₁₂-Alkylphenylsulfonat oder Mono-C₁-C₄-alkylsulfat oder L¹ und L² können gemeinsam eine Brücke -O- darstellen.
   Die Reaktionsfolge IIa → IIb → IIc kann zweistufig oder vorzugsweise einstufig, d.h. ohne Isolierung der im ersten Verfahrensschritt gebildeten Hydroxamsäure IIb hergestellt werden. Die Durchführung kann analog den in WO 95/04728, WO 97/00866 und EP-A 846 691 beschriebenen Methoden ausgeführt werden.
   Verbindungen der Formel Va sind literaturbekannt oder lassen sich nach bekannten Methoden herstellen [vgl.: WO-A 95/04728; WO-A 97/00866; EP-A 846 691].
   Benzylverbindungen der Formel IIa sind ebenfalls literaturbekannt oder können nach bekannten Methoden hergestellt werden [vgl.: EP-A 178 826; EP-A 253 213; WO-A 95/04728]. 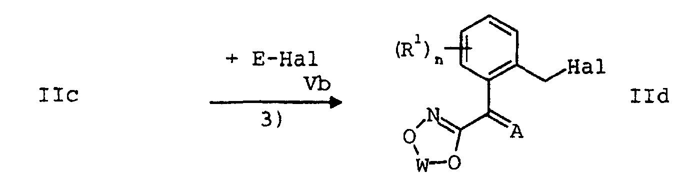
3) Verbindungen II, in denen L für Halogen steht (Formel IId), werden aus Verbindungen IIc durch Umsetzung mit einem Säurehalogenid oder einer Halogenwasserstoffsäure E-Hal (Formel IVb) gegebenenfalls in Gegenwart einer Lewis-Säure erhalten [vgl.: Jp-A 90/89653; DE-A 29 33 985; WO-A 95/04728; Tetrahedron Lett., Bd. 34, S. 3829 (1993)]. Es kann vorteilhaft sein, zuvor in einem separaten Schritt die Schutzgruppe der Verbindung IIc abzuspalten [vgl. T. W. Greene, Protective Groups in Organic Chemistry, J. Wiley & Sons, 1991, S. 10 - 142].
   Alternativ kann aus Verbindung IIc zunächst die Schutzgruppe abgespalten werden und anschließend der entstandene Benzylalkohol durch Umsetzung mit üblichen Halogenierungsmitteln, wie beispielsweise SOCl₂, BBr₃/SnCl₄, CCl₄/Triphenylphosphin oder HBr, in Verbindung IId überführt werden [vgl.: Organikum, 16. Auflage 1988, S. 189ff., VEB Verlag der Wissenschaften, Berlin; Synthesis (1989) S. 614; J. Org. Chem., Bd. 53 (1988) S. 5113; Synth. Commun., Bd. 17 (1987) S. 219].
   In Formel Vb steht E für C₁-C₄-Alkylcarbonyl, Arylcarbonyl, C₁-C₄-Alkylsulfonyl oder Arylsulfonyl und Hal für Halogen (z.B. Chlor oder Brom). Für Verbindungen Vb kommen insbesondere Carbonsäurechloride, wie z.B. Essigsäurechlorid in Betracht. Als bevorzugte Lewis-Säure sei Aluminiumtrichlorid beispielhaft genannt.
   Verbindungen IId sind auch aus o-Tolylverbindungen der Formel IIa, in der L^{a} für Wasserstoff steht, durch radikalische Halogenierung unter allgemein üblichen Bedingungen zugänglich [vgl. J. Amer. Chem. Soc., Bd. 71, S. 2137ff. (1949); ebd., Bd. 90, S. 1797ff. (1968)]. 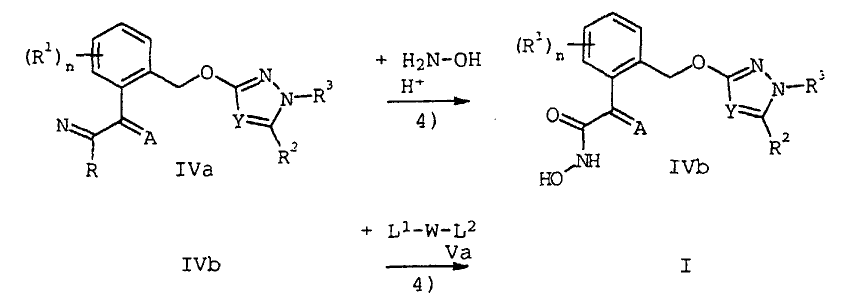
4) Alternativ sind Verbindungen der Formel I auch aus Verbindungen der Formel IVa zugänglich. Sie können über die Reaktionsfolge IVa → IVb → I analog der in Abschnitt 2) genannten Bedingungen in Verbindungen der Formel I überführt werden.

In Formel IVa haben der Substituent R die für Formel IIa und die anderen Reste die für Formel I definierte Bedeutung.

Verbindungen IVa sind bekannt, bzw. nach bekannten Methoden zugänglich [vgl.: EP-A 378 755, EP-A 757 042, WO-A 94/19331, WO-A 95/29896 und WO-A 96/37480].

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Die Verbindungen I können bei der Herstellung aufgrund ihrer C=Cund C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende UmWandlungen können auch nach der Anwendung, beispielsweise bei der Behandlung von Pflanzen in der behandelten Pflanze oder im zu bekämpfenden Schadpilz oder tierischen Schädling erfolgen.

Bei den in den vorstehenden Formeln angegebenen Definitionen der Symbole wurden Sammelbegriffe verwendet, die allgemein repräsentativ für die folgenden Substituenten stehen:
- Halogen: Fluor, Chlor, Brom und Jod;
- C₁-C₄-Alkyl sowie die Akylteile von C₁-C₄-Alkoxy, C₁-C₄-Alkylthio, C₁-C₄-Alkylamino, Di-C₁-C₄-alkylamin oder C₁-C₄-Alkylcarbonyl -oxy: gesättigte, geradkettige oder verzweigte Kohlenwasserstoffreste mit 1 bis 4 Kohlenstoffatomen, im einzelnen Methyl, Ethyl, Propyl, 1-Methylethyl, Butyl, 1-Methyl-propyl, 2-Methyl-propyl, 1,1-Dimethylethyl;
- C₁-C₆-Alkyl: gesättigte, geradkettige oder verzweigte Kohlenwasserstoffreste mit 1 bis 6 Kohlenstoffatomen, z.B. C₁-C₄-Alkyl wie vorstehend genannt oder Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
- C₁-C₃-Alkylen: Methylen, Ethylen oder n-Propylen;
- C₁-C₄-Halogenalkyl sowie die Halogenalkylteile von C₁-C₄-Halogen -alkoxy: geradkettige oder verzweigte Alkylgruppen mit 1 bis 4 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
- C₃-C₄-Alkenyl: ungesättigte, geradkettige oder verzweigte Kohlenwasserstoffreste mit 3 bis 4 Kohlenstoffatomen und einer Doppelbindung in einer beliebigen Position, z.B. 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl und 2-Methyl-2-propenyl;
- C₂-C₄-Alkenyl sowie die Alkenylteile von C₂-C₄-Alkenoxy: Ethenyl oder C₃-C₄-Alkenyl (wie vorstehend genannt);
- C₂-C₄-Halogenalkenyl sowie die Halogenalkenylteile von C₂-C₄-Halogenalkenoxy: ungesättigte, geradkettige oder verzweigte Kohlenwasserstoffreste mit 2 bis 4 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
- C₃-C₄-Alkinyl: geradkettige oder verzweigte Kohlenwasserstoffgruppen mit 3 bis 4 Kohlenstoffatomen und einer Dreifachbindung in einer beliebigen Position, z.B. Ethinyl, 1-Propinyl, 2-Propinyl, 1-Butiny 2-Butinyl, 3-Butinyl und 1-Methyl-2-propinyl;
- C₂-C₄-Alkinyl sowie die Alkinylteile von C₂-C₄-Alkinoxy: Ethinyl oder C₃-C₄-Alkinyl (wie vorstehend genannt);
- C₃-C₄-Halogenalkinyl sowie die Halogenalkinylteile von C₂-C₄-Halogenalkinoxy: ungesättigte, geradkettige oder verzweigte Kohlenwasserstoffreste mit 2 bis 4 Kohlenstoffatomen und einer Dreifachbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
Aryl: ein ein- bis dreikerniges aromatisches Ringsystem enthaltend 6 bis 14 Kohlenstoffringglieder, z.B. Phenyl, Naphthyl und Anthracenyl;
Hetaryl: 5- oder 6-gliedrige Heteroaromaten, z.B.
- 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
- benzokondensiertes 5-gliedriges Heteroaryl, enthaltend ein bis drei Stickstoffatome oder ein Stickstoffatom und ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
- 6-gliedriges Heteroaryl, enthaltend ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;

Der Zusatz "*ggf. subst*" in Bezug auf den Phenylrest soll zum Ausdruck bringen, daß dieser Rest partiell oder vollständig halogeniert sein kann [d.h. die Wasserstoffatome dieses Restes können teilweise oder vollständig durch gleiche oder verschiedene Halogenatome wie vorstehend genannt (vorzugsweise Fluor, Chlor oder Brom, insbesondere Fluor oder Chlor) ersetzt sein] und/oder einen bis vier (insbesondere einen bis drei) der folgenden Reste tragen können:
Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-alkylamino und C₁-C₄-Alkylthio.

Im Hinblick auf ihre bestimmungsgemäße Verwendung sind Azadioxacycloalkene der Formel I mit folgenden Substituenten bevorzugt, wobei die Bevorzugung jeweils für sich allein oder in Kombination zu sehen ist:

Die besonders bevorzugten Ausführungsformen der Zwischenprodukte. in Bezug auf die Variablen entsprechen denen der Reste Y, n, A, W, R¹, R² und R³ der Formel I.

Insbesondere werden Verbindungen I' bevorzugt.

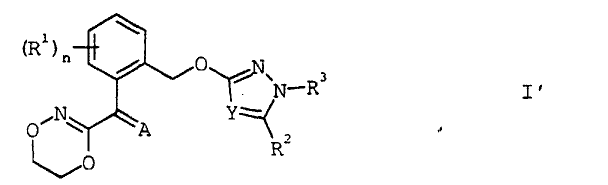

Außerdem werden Verbindungen I besonders bevorzugt, in denen A für =N-OCH₃, =CH-OCH₃ oder =CH-CH₃ steht.

Insbesondere werden Verbindungen I bevorzugt, in denen A für =N-OR⁴ oder CH-R⁴ steht.

Darüber hinaus werden Verbindungen I besonders bevorzugt, in denen A für =N-OCH₃ oder =CH-CH₃ steht.

Daneben werden Verbindungen I besonders bevorzugt, in denen n für Null steht.

Gleichermaßen besonders bevorzugt sind Verbindungen I, in deren R¹ für 6-CH₃ oder 6-Cl und n für 1 steht.

Insbesondere werden auch Verbindungen I bevorzugt, in denen Y für N steht.

Außerdem werden Verbindungen I besonders bevorzugt, in denen Y für CR^{a} steht.

Gleichermaßen besonders bevorzugt sind Verbindungen I, in deren R² für Wasserstoff steht.

Daneben werden Verbindungen I besonders bevorzugt, in denen R³ für C₁-C₆-Alkyl steht, das unsubstituiert oder partiell oder vollständig halogeniert ist oder eine bis drei Gruppen R^{b} tragen kann.

Desweiteren werden Verbindungen I besonders bevorzugt, in denen R³ für Phenyl oder Benzyl steht, wobei der Phenylring partiell oder vollständig halogeniert sein oder eine bis drei Gruppen R^{c} tragen kann.

Außerdem werden Verbindungen I besonders bevorzugt, in denen R³ für Pyridin-2-yl steht, welches partiell oder vollständig halogeniert sein oder eine bis drei Gruppen R^{c} tragen kann.

Insbesondere werden Verbindungen IA bevorzugt, in denen Y für N, CH oder C-CH₃, (R¹)ₙ für Wasserstoff, 6-CH₃ oder 6-Cl, R² für Wasserstoff oder CH₃ und R³ für Cyclohexyl, Benzyl, Phenyl oder 2-Pyridyl steht, wobei die aromatischen Ringe durch eine oder zwei Gruppen Halogen oder Trifluormethyl substituiert sein können.

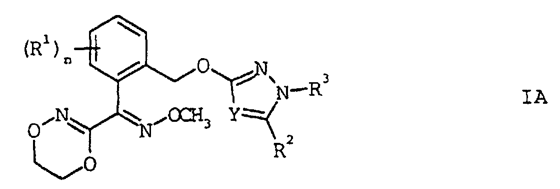

Außerdem werden Verbindungen IB bevorzugt, in denen Y für N, CH oder C-CH₃, (R¹)ₙ für Wasserstoff, 6-CH₃ oder 6-Cl, R² für Wasserstoff oder CH₃ und R³ für Cyclohexyl, Benzyl, Phenyl oder 2-Pyridyl steht, wobei die aromatischen Ringe durch eine oder zwei Gruppen Halogen oder Trifluormethyl substituiert sein können.

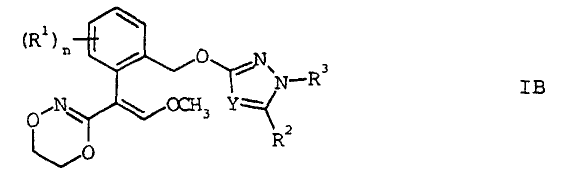

Gleichermaßen werden Verbindungen IC bevorzugt, in denen Y für N, CH oder C-CH₃, (R¹)ₙ für Wasserstoff, 6-CH₃ oder 6-Cl, R² für Wasserstoff oder CH₃ und R³ für Cyclohexyl, Benzyl, Phenyl oder 2-Pyridyl steht, wobei die aromatischen Ringe durch eine oder zwei Gruppen Halogen oder Trifluormethyl substituiert sein können.

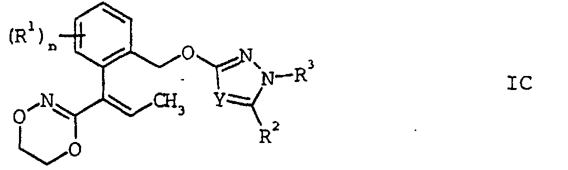

Daneben werden Verbindungen ID besonders bevorzugt, in denen (R¹)ₙ für Wasserstoff, 6-CH₃ oder 6-Cl, Y für N, CH oder C-CH₃, R² für Wasserstoff, CH₃ oder Chlor, R^{c} für Halogen, Nitro, Cyano, C₁-C₄-Alkyl, C₁-C₆-Alkoxy, C₁-C₂-Halogenalkoxy, Phenyl-C₁-C₂-alkoxy, C(CH₃)=NO-C₁-C₃-Alkyl, Cyclohexyl, Phenyl oder Pyridyl und p für 0, 1 oder 2 steht.

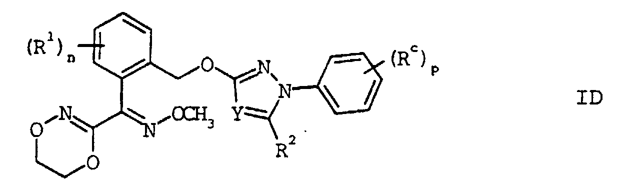

Insbesondere werden auch Verbindungen IE bevorzugt, in denen (R¹)ₙ für Wasserstoff, 6-CH₃ oder 6-Cl, Y für N, CH oder C-CH₃, R² für Wasserstoff, CH₃ oder Chlor, R^{c} für Halogen, Nitro, Cyano, C₁-C₄-Alkyl, C₁-C₆-Alkoxy, C₁-C₂-Halogenalkoxy, Phenyl-C₁-C₂-alkoxy, C(CH₃)=NO-C₁-C₃-Alkyl, Cyclohexyl, Phenyl oder Pyridyl und p für 0, 1 oder 2 steht.

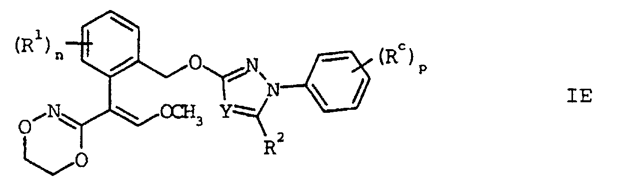

Außerdem werden Verbindungen IF besonders bevorzugt, in denen (R¹)ₙ für Wasserstoff, 6-CH₃ oder 6-Cl, Y für N, CH oder C-CH₃, R² für Wasserstoff, CH₃ oder Chlor, R^{c} für Halogen, Nitro, Cyano, C₁-C₄-Alkyl, C₁-C₆-Alkoxy, C₁-C₂-Halogenalkoxy, Phenyl-C₁-C₂-alkoxy, C(CH₃)=NO-C₁-C₃-Alkyl, Cyclohexyl, Phenyl oder Pyridyl und p für 0, 1 oder 2 steht.

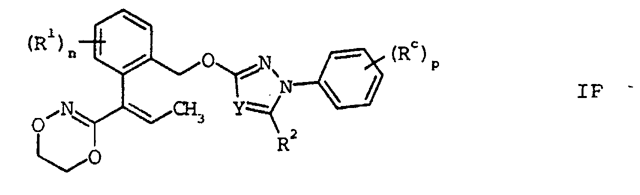

Gleichermaßen besonders bevorzugt sind Verbindungen IG, in denen (R¹)ₙ für Wasserstoff, 6-CH₃ oder 6-Cl, Y für N, CH oder C-CH₃, R² für Wasserstoff, CH₃ oder Chlor, R^{c} für Halogen, Methyl, Trifluormethyl, Methoxy oder Trifluormethoxy und p für 0 oder 1 steht.

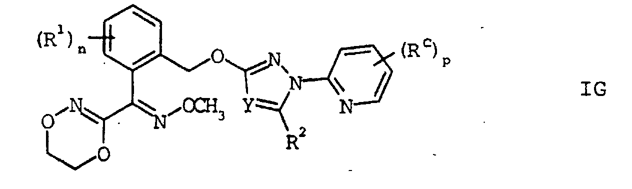

Daneben werden Verbindungen IH besonders bevorzugt, in denen (R¹)ₙ für Wasserstoff-, 6-CH₃ oder 6-Cl, Y für N, CH oder C-CH₃, R² für Wasserstoff, CH₃ oder Chlor, R^{c} für Halogen, Methyl, Trifluormethyl, Methoxy oder Trifluormethoxy und p für 0 oder 1 steht.

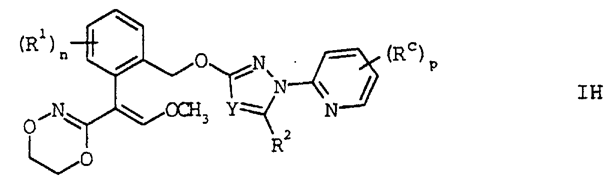

Desweiteren werden Verbindungen IJ besonders bevorzugt, in denen (R¹)ₙ für Wasserstoff, 6-CH₃ oder 6-Cl, Y für N, CH oder C-CH₃, R² für Wasserstoff, CH₃ oder Chlor, R^{c} für Halogen, Methyl, Trifluormethyl, Methoxy oder Trifluormethoxy und p für 0 oder 1 steht.

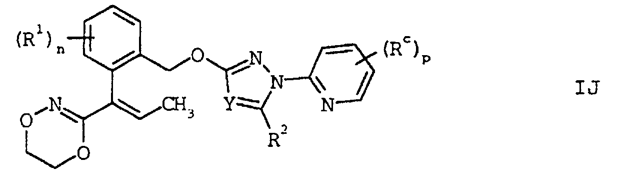

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen und Erdnüssen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudoperonospora-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen der Formel I sind außerdem geeignet, tierische Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor zur Bekämpfung tierischer Schädlinge eingesetzt werden. Insbesondere eignen sie sich zur Bekämpfung der folgenden tierischen Schädlinge:
- Insekten aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis,
- Käfer (Coleoptera), z.B. Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum,- Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria,
- Zweiflügler (Diptera), z.B. Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea und Tipula paludosa,
- Thripse (Thysanoptera), z.B. Frankliniella fusca, Frankliniella occidentalis, Frankliniella tritici, Scirtothrips citri, Thrips oryzae, Thrips palmi und Thrips tabaci,
- Hautflügler (Hymenoptera), z.B. Athalia rosae, Atta cephalotes, Atta sexdens, Atta texana, Hoplocampa minuta, Hoplocampa testudinea, Monomorium pharaonis, Solenopsis geminata und Solenopsis invicta,
- Wanzen (Heteroptera), z.B. Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis und Thyanta perditor,
- Pflanzensauger (Homoptera), z.B. Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum und Viteus vitifolii,
- Termiten (Isoptera), z.B. Calotermes flavicollis, Leucotermes flavipes, Reticulitermes lucifugus und Termes natalensis,
- Geradflügler (Orthoptera), z.B. Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus und Tachycines asynamorus,
- Arachnoidea wie Spinnentiere (Acarina), z.B. Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius und Tetranychus urticae,
- Nematoden wie Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus und Pratylenchus goodeyi.

Die Aufwandmenge an Wirkstoff zur Bekämpfung von tierischen Schädlingen beträgt unter Freilandbedingungen 0,1 bis 2,0, vorzugsweise 0,2 bis 1,0 kg/ha.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylenbis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Puryl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-ylethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl-ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-(3,5-Dichlorphenyl)-5-methyl-5-methoxymethyl-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1 Synthese von (5,6-Dihydro-[1,4,2]dioxazin-3-yl)-(2-ortho-tolyloxymethyl)-phenyl-methanon-O-methyloxim

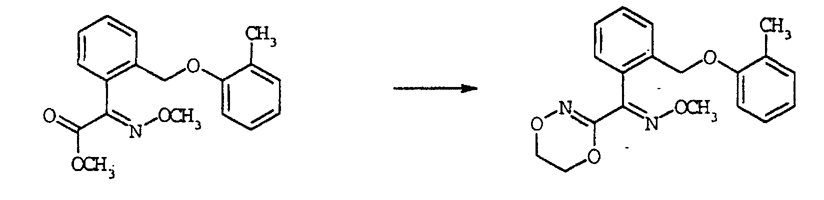

Zu einer Lösung von 59,4 g Kaliumhydroxid (85 %ig) in 270 ml wasserfr. Methanol wurde eine Lösung von 62,7 g Hydroxylammoniumchlorid in 570 ml wasserfr. Methanol sowie 93,9 g Methyl-(E)-methoximino[α-(ortho-toloyloxy)-ortho-tolyl]acetat [vgl.: EP-A 253 213] gegeben und 4 Std. refluxiert. Dann wurden 141 g 1,2-Dibromethan und 82,8 g Kaliumcarbonat zugegeben weitere 14 Std. unter Rückfluß gerührt. Dann wurde die Reaktionsmischung vom Lösungsmittel befreit, der Rückstand in Ethylacetat aufgenommen, mit Wasser gewaschen und getrocknet. Nach Abdestillieren des Lösungsmittels und Chromatographie an Kieselgel (Cyclohexan-Ethylacetat-Gemisch [1:1]) erhielt man 43,7 g des Produktes als farbloses amorphes Pulver vom Fp 104-106°C.

### Beispiel 2 Synthese von (ortho-Chlormethylphenyl)-(5,6-dihydro-[1,4,2]dioxazin-3-yl)-methanon-O-methyl-oxim

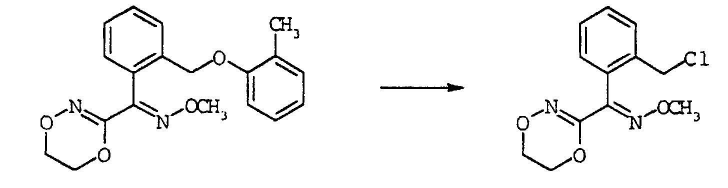

Zu einer Suspension von 5,2 g Aluminiumtrichlorid in 50 ml wasserfr. Dichlormethan wurden 3,66 g Acetylchlorid getropft. Anschließend wurde eine Lösung von 5,3 g des Oxims aus Beispiel 1 in 20 ml wasserfr. Dichlormethan zugetropft und 3 Std. bei 20 bis 25 °C gerührt. Nach Verdünnen mit 100 ml Dichlormethan wurde die Reaktionslösung auf Eiswasser gegossen. Nach Phasentrennung wurde die organische Phase mit Wasser gewaschen, getrocknet und vom Lösungsmittel befreit. Nach Chromatographie an Kieselgel (Cyclohexan-Ethylacetat-Gemisch [1:1]) erhielt man 3,14 g der Titelverbindung als hellbeige Kristalle vom Fp. 93-96 °C.

### Beispiel 3 Synthese von (ortho-[1-{4-Chlorphenyl}-pyrazol-3-yloxymethyl]-phenyl)-(5,6-dihydro-[1,4,2]dioxazin-3-yl)-methanon-O-methyl-oxim

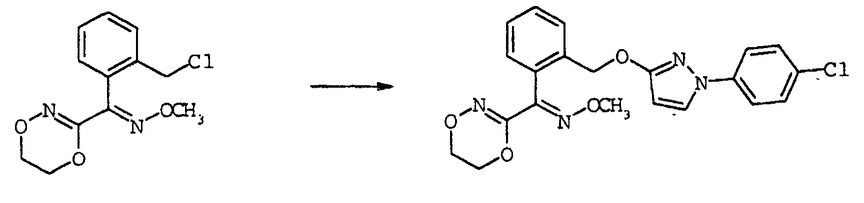

Die Lösung von 0,97 g 1-(para-Chlorphenyl)-3-hydroxy-pyrazol in 20 wasserfr. Dimethylformamid (DMF) wurde nach Versetzen mit 0,13 g Natriumhydrid etwa eine Std. bei 20 bis 25 °C gerührt. Dann wurden 1,34 g des Oxims aus Beispiel 2 zugegeben, die Mischung etwa drei Std. bei 60 °C und dann noch etwa 14 Std. bei 20 bis 25 °C gerührt. Nach zusetzen von 300 ml Wasser wurde mit Methyltert.Butylether (MTBE) extrahiert. Die vereinigte organ. Phase wurde nach Waschen mit Wasser und Trocknen vom Lösungsmittel befreit. Nach Chromatographie des Rückstandes an Kieselgel (Cyclohexan-Ethylacetat-Gemisch [1:1]) erhielt man 1,15 g der Titelverbindung als hellbeiges amorphes Pulver vom Fp. 56-59 °C.

IR [cm-1]: 1546, 1502, 1480, 1464, 1358, 1093, 1054, 998, 935, 906.

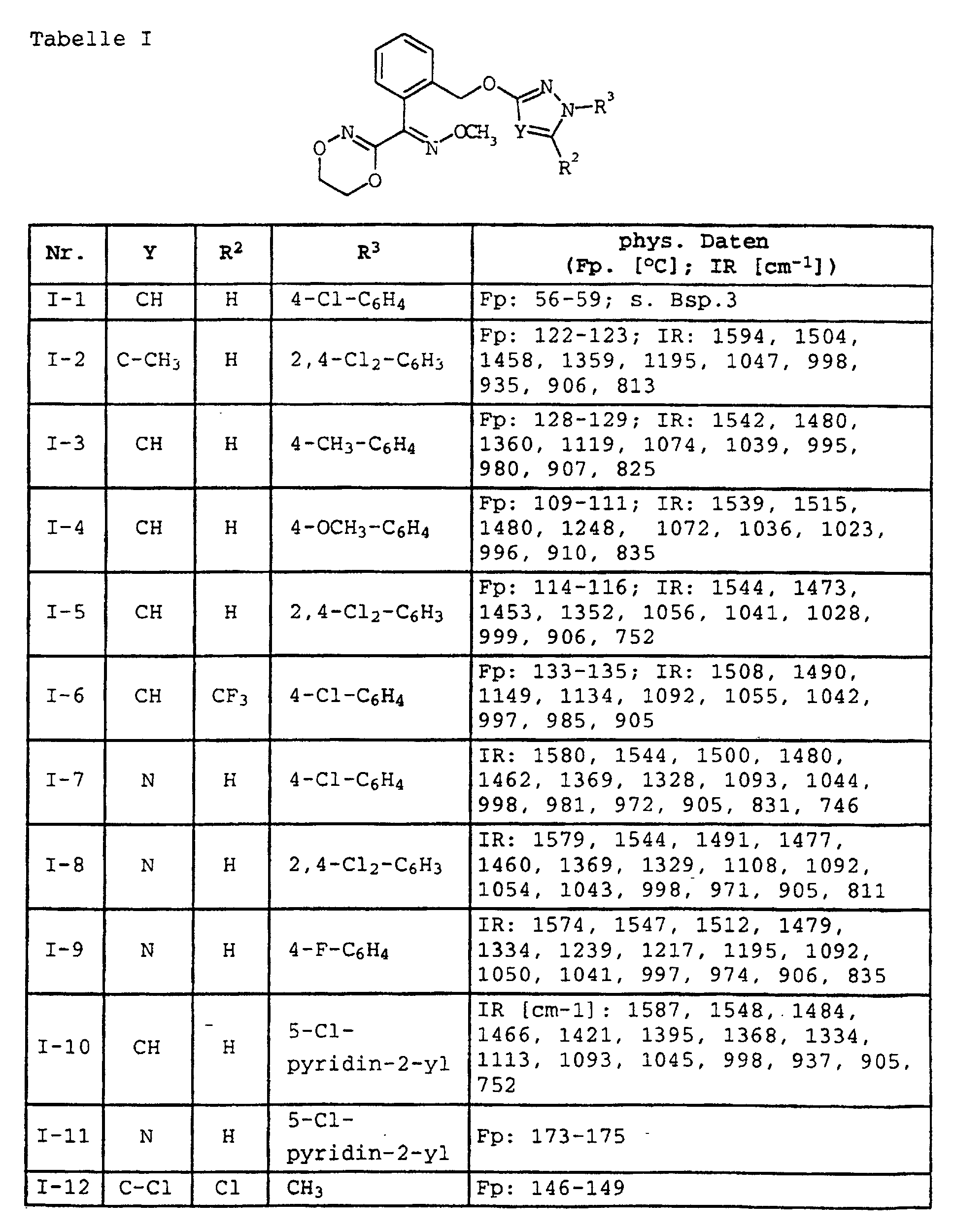

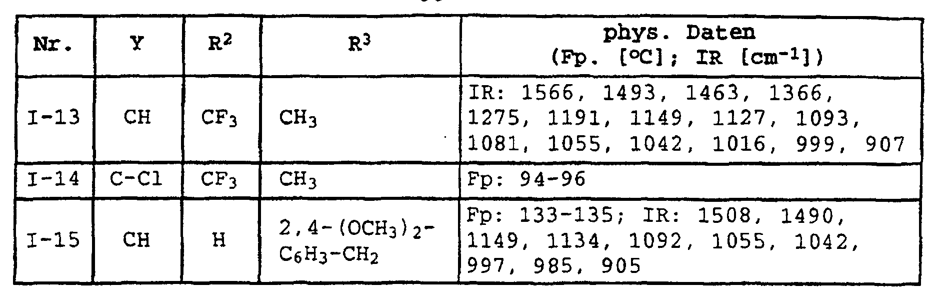

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ig Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

Als Vergleichswirkstoffe dienten die aus EP-A 757 042 (Nr. 20 der Tabelle 2; Verbindung A), bzw. aus WO-A 95/04728 (Nr. 58 der Tabelle 1; Verbindung B) bekannten Substanzen:

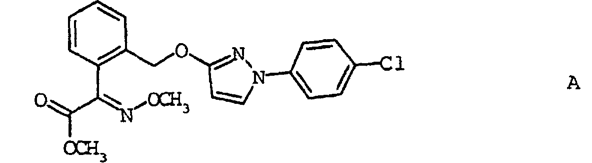

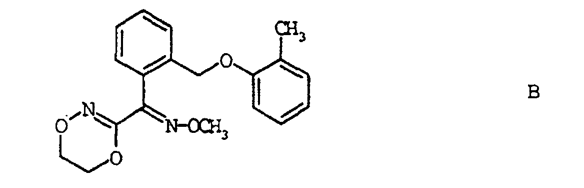

Anwendungsbeispiel 1 - Kurative Wirksamkeit gegen Puccinia recondita an Weizen (Weizenbraunrost)

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden in eine Kammer mit hoher Luftfeuchtigkeit (90 bis 95%) und 20 bis 22°C gestellt. Während dieser Zeit keimten die Sporen aus und die Keimschläuche drangen in das Blattgewebe ein. Die infizierten Pflanzen wurden am nächsten Tag mit einer wäßrigen Wirkstoffaufbereitung, die aus einer Stammlösung bestehend aus 10% Wirkstoff, 63% Cyclohexanon und 27% Emulgiermittel angesetzt worden war, tropfnaß besprüht. Nach dem Antrocknen des Spritzbelages wurden die Versuchspflanzen im Gewächshaus bei Temperaturen zwischen 20 und 22°C und 65 bis 70% relativer Luftfeuchte für 7 Tage kultiviert. Dann wurde das Ausmaß der Rostpilzentwicklung auf den Blättern ermittelt.

In diesem Test zeigten die mit 16 ppm der Verbindungen I-1, I-2, I-4 und I-10 behandelten Pflanzen keinen Befall, während die mit 16 ppm der Vergleichswirkstoffe A und B behandelten Pflanzen zu 60%, bzw. 90% und die unbehandelten zu 100% befallen waren.

### Anwendungsbeispiel 2 - Wirksamkeit gegen Botrytis cinerea an Paprikablättern

Paprikasämlinge der Sorte "Neusiedler Ideal Elite" wurden, nachdem sich 4 bis 5 Blätter gut entwickelt hatten, mit einer wäßrigen Wirkstoffaufbereitung, die aus einer Stammlösung aus 10% Wirkstoff, 63% Cyclohexanon und 27% Emulgiermittel angesetzt wurde, bis zur Tropfnässe besprüht. Am nächsten Tag wurden die behandelten Pflanzen mit einer Sporensuspension von Botrytis cinerea, die 1,7 x 10⁶ Sporen/ml in einer 2%igen wäßrigen Biomalzlösung enthielt, inokuliert. Anschließend wurden die Versuchspflanzen in eine Klimakammer mit 22 bis 24°C und hoher Luftfeuchtigkeit gestellt. Nach 5 Tagen konnte das Ausmaß des Pilzbefall auf den Blättern visuell in % ermittelt werden.

In diesem Test zeigten die mit 250 ppm der Verbindung I-1 behandelten Pflanzen 3% Befall, während die mit 250 ppm des Vergleichswirkstoffs A behandelten Pflanzen zu 40% und die unbehandelten zu 80% befallen waren.

Anwendungsbeispiel 3 - Wirksamkeit gegen Pyricularia oryzae (protektiv)

Blätter von in Töpfen gewachsenen Reiskeimlingen der Sorte "Tai-Nong 67" wurden mit wäßriger Wirkstoffaufbereitung, die mit einer Stammlösung aus 10% Wirkstoff, 63% Cyclohexanon und 27% Emulgiermittel angesetzt wurde, bis zur Tropfnässe besprüht. Am folgenden Tag wurden die Pflanzen mit einer wäßrigen Sporensuspension von Pyricularia oryzae inokuliert. Anschließend wurden die Versuchspflanzen in Klimakammern bei 22 bis 24°C und 95 bis 99% relativer Luftfeuchtigkeit für 6 Tage aufgestellt. Dann wurde das Ausmaß der Befallsentwicklung auf den Blättern visuell ermittelt.

In diesem Test zeigten die mit 16 ppm der Verbindungen I-1, I-2 und I-10 behandelten Pflanzen 10% Befall, während die mit 16 ppm des Vergleichswirkstoffs B behandelten Pflanzen zu 40% und die unbehandelten zu 90% befallen waren.

Anwendungsbeispiel 4 - Wirksamkeit gegen Wirksamkeit gegen Phytophthora infestans an Tomaten

Blätter von Topfpflanzen der Sorte "Große Fleischtomate St. Pierre" wurden mit einer wäßrigen Suspension, die aus einer Stammlösung aus 10% Wirkstoff, 63% Cyclohexanon und 27% Emulgiermittel angesetzt wurde, bis zur Tropfnässe besprüht. Am folgenden Tag wurden die Blätter mit einer kalten wäßrigen Zoosporenaufschwemmung von Phytophthora infestans mit einer Dichte von 0,25 x 10⁶ Sporen/ml infiziert. Anschließend wurden die Pflanzen in einer wasserdampfgesättigten Kammer bei Temperaturen zwischen 18 und 20°C aufgestellt. Nach 6 Tagen hatte sich die Krautfäule auf den unbehandelten, jedoch infizierten Kontrollpflanzen so stark entwickelt, daß der Befall visuell in % ermittelt werden konnte.

In diesem Test zeigten die mit 250 ppm der Verbindungen I-4, I-10, I-12 und I-14 behandelten Pflanzen maximal 5% Befall, während die unbehandelten Pflanzen zu 90% befallen waren.

### Beispiele für die Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollen noch eine 80 bis 100%-ige Hemmung bzw. Mortalität hervorriefen (Wirkschwelle bzw. Minimalkonzentration).

## Patentansprüche

1. Azadioxacycloalkene der Formel I 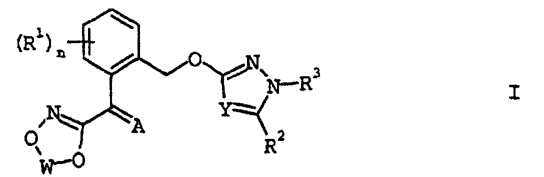 in der die Substituenten und der Index die folgende Bedeutung haben:
Y N oder CR^{a}, wobei
R^{a} für Wasserstoff, Halogen oder C₁-C₄-Alkyl steht;
n 0, 1, 2, 3 oder 4, wobei die Substituenten R¹ verschieden sein können, wenn n größer als 1 ist;
R¹ Nitro, Cyano, Halogen, C₁-C₆-Alkyl oder
für den Fall, daß n größer 1 ist, zusätzlich eine an zwei benachbarte Ringatome gebundene Brücke, welche drei bis vier Glieder ausgewählt aus der Gruppe: 3 oder 4 Kohlenstoffatome, 2 bis 3 Kohlenstoffatome und 1 oder 2 Stickstoff-, Sauerstoff- und/oder Schwefelatome enthält, wobei diese Brücke gemeinsam mit dem Ring an den sie gebunden ist einen partiell ungesättigten oder aromatischen Rest bilden kann und weiterhin die Kohlenstoffatome der Brücke durch Halogenatome oder Methylgruppen teilweise oder vollständig substituiert sein können;
R² Wasserstoff, Nitro, Cyano, Halogen, C₁-C₄-Alkyl, C₁-C₄-Halogenalkyl, C₁-C₄-Alkoxy, C₁-C₄-Alkylthio oder C₁-C₄-Alkoxycarbonyl;
R³ C₁-C₆-Alkyl, das unsubstituiert oder partiell oder vollständig halogeniert ist oder eine bis drei Gruppen R^{b} tragen kann,
R^{b} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy und C₁-C₄-Alkylendioxy, welches halogeniert sein kann,
C₃-C₆-Cycloalkyl, Phenyl, Naphthyl, 5-gliedriges Heteroaryl, enthaltend ein bis vier Stickstoffatome oder ein bis drei Stickstoffatome und ein Schwefel- oder Sauerstoffatom oder 6-gliedriges Heteroaryl, enthaltend ein *bis* vier Stickstoffatome,
wobei die cyclischen Systeme partiell oder vollständig halogeniert sein oder eine bis drei Gruppen R^{c} tragen können:
R^{c} Halogen, Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyl- oder Alkinylgruppen in diesen Resten 2 bis 8 Kohlenstoffatome enthalten;
C(=NOR^{d})-Γ₁-R^{d'}, wobei R^{d} für Wasserstoff oder C₁-C₆-Alkyl, Γ für Sauerstoff, Schwefel oder NR^{d} steht und 1 gleich 0 oder 1 ist;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Phenyl, Phenoxy, Phenylthio, Phenyl-C₁-C₆-alkoxy, Phenyl-C₁-C₆-alkyl, Hetaryl, Hetaryloxy, Hetarylthio, wobei die Hetarylreste 5 oder 6 Ringglieder enthalten, wobei die cyclischen Systeme partiell oder vollständig halogeniert oder durch eine bis drei Gruppen R^{b} substituiert sein können;
A =N-OR⁴, =CH-OR⁴ =CH-SR⁴ oder =CH-R⁵, wobei
R⁴ C₁-C₄-Alkyl oder C₁-C₄-Halogenalkyl,
R⁵ Halogen oder eine Gruppe R⁴ bedeutet und
W C₁-C₃-Alkylen, das unsubstituiert oder durch eine oder zwei Gruppen R^{e} substituiert ist, wobei
R^{e} für Halogen, Cyano, C₁-C₄-Alkyl, C₁-C₄-Halogenalkyl, C₂-C₄-Alkenyl, C₂-C₄-Halogenalkenyl, C₂-C₄-Alkinyl oder C₂-C₄-Halogenalkinyl, C₁-C₄-Alkoxy, C₁-C₄-Halogenalkoxy, C₂-C₄-Alkenoxy, C₂-C₄-Halogen-alkenoxy, C₂-C₄-Alkinoxy oder C₂-C₄-Halogenalkinoxy, C₁-C₄-Alkylcarbonyloxy steht.

2. Azadioxacycloalkene der Formel I'. 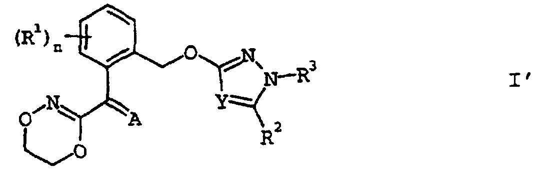 in der
R¹ 6-Cl oder 6-CH₃;
n 0 oder 1;
A =N-OCH₃, =CH-OCH₃ oder =CH-CH₃ bedeuten und
Y, R² und R³ die in Anspruch 1 gegebene Bedeutung haben.

3. Verfahren zur Herstellung der Verbindungen der Formel I nach Anspruch 1, **dadurch gekennzeichnet, daß** man Benzylhalogenide der Formel II, 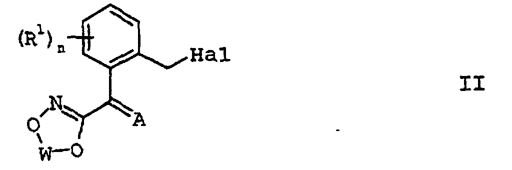 in der R¹, n, A und W die in Anspruch 1 gegebene Bedeutung haben und Hal für ein Halogenatom steht, mit Verbindungen der Formal III, 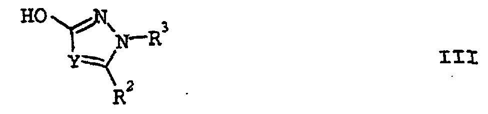 in der Y, R² und R³ die in Anspruch 1 gegebene Bedeutung haben, in Gegenwart einer Base umsetzt.

4. Verfahren zur Herstellung der Verbindungen der Formel I nach Anspruch 1, **dadurch gekennzeichnet, daß** man Phenylessigsäurederivate der Formel IVa, 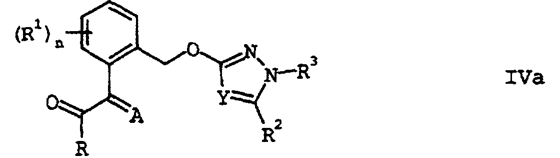 in der R¹, n, R², R³ und A die in Anspruch 1 gegebene Bedeutung haben und R für Halogen, C₁-C₆-Alkylcarbonyloxy, OH, C₁-C₄-Alkoxy, Phenoxy oder Azido steht, gegebenenfalls in Gegenwart einer Base und/oder eines Kupplungsreagenzes mit Hydroxylamin oder dessen Säureadditionssalzen zu Hydroxamsäuren der Formel IVb, 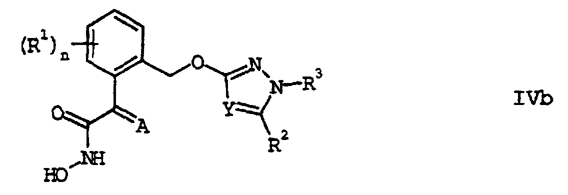 und IVb mit Verbindungen der Formel IVa,
L¹-W-L² Va
in der W die in Anspruch 1 genannte Bedeutung hat und L¹, L² jeweils für eine nucleophil austauschbare Gruppe stehen, gegebenenfalls in Gegenwart einer Base oder eines wasserentziehenden Mittels umsetzt.

5. Zwischenprodukte der Formel IVb gemäß Anspruch 4.

6. Zur Bekämpfung von tierischen Schädlingen oder Schadpilzen geeignetes Mittel, enthaltend einen festen oder flüssigen Trägerstoff und eine Verbindung der allgemeinen Formel I gemäß Anspruch 1.

7. Verwendung der Verbindungen I gemäß Anspruch 1 zur Herstellung eines zur Bekämpfung von tierischen schädlingen oder Schadpilzen geeigneten Mittels.

8. Verfahren zur Bekämpfung von Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der allgemeinen Formel I gemäß Anspruch 1 behandelt.

9. Verfahren zur Bekämpfung von tierischen Schädlingen, **dadurch gekennzeichnet, daß** man die tierischen Schädlinge oder die vor ihnen zu schützenden Materialien, Pflanzen, den Boden oder Saatgüter mit einer wirksamen Menge einer Verbindung der allgemeinen Formel I gemäß Anspruch 1 behandelt.

## Claims

1. An azadioxacycloalkene of the formula I 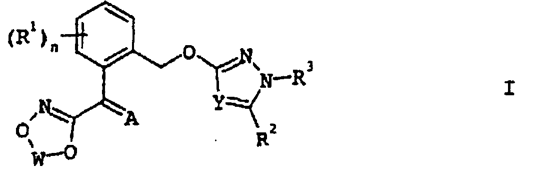 where the substituents and the index have the following meanings:
Y is N or CR^{a}, where
R^{a} is hydrogen, halogen or C₁-C₄-alkyl;
n is 0, 1, 2, 3 or 4, where the substituents R¹ may be different if n is greater than 1;
R¹ is nitro, cyano, halogen, C₁-C₆-alkyl or,
if n is greater than 1, additionally a bridge which is attached to two adjacent ring atoms and which contains three to four members selected from the group consisting of: 3 or 4 carbon atoms, 2 to 3 carbon atoms and 1 or 2 nitrogen, oxygen and/or sulfur atoms, where this bridge, together with the ring to which it is attached, may form a partially unsaturated or aromatic ring and where furthermore the carbon atoms of the bridge may be partially or fully substituted by halogen atoms or methyl groups;
R² is hydrogen, nitro, cyano, halogen, C₁-C₄-alkyl, C₁-C₄-haloalkyl, C₁-C₄-alkoxy, C₁-C₄-alkylthio or C₁-C₄-alkoxycarbonyl;
R³ is C₁-C₆-alkyl which is unsubstituted or partially or fully halogenated or may carry one to three groups R^{b},
R^{b} is halogen, cyano, nitro, hydroxyl, C₁-C₆-alkyl, C₁-C₆-haloalkyl, C₁-C₆-alkylcarbonyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyl, C₂-C₆-alkenyloxy, C₃-C₆-alkynyloxy and C₁-C₄-alkylenedioxy, which may be halogenated,
C₃-C₆-cycloalkyl, phenyl, naphthyl, 5-membered heteroaryl containing one to four nitrogen atoms or one to three nitrogen atoms and one sulfur or oxygen atom, or 6-membered heteroaryl containing one to four nitrogen atoms,
where the cyclic systems may be partially or fully halogenated or may carry one to three groups R^{c}:
R^{c} is halogen, cyano, nitro, hydroxyl, mercapto, amino, carboxyl, aminocarbonyl, aminothiocarbonyl, alkyl, haloalkyl, alkenyl, alkenyloxy, alkynyloxy, alkoxy, haloalkoxy, alkylthio, alkylamino, dialkylamino, formyl, alkylcarbonyl, alkylsulfonyl, alkylsulfoxyl, alkoxycarbonyl, alkylcarbonyloxy, alkylaminocarbonyl, dialkylaminocarbonyl, alkylaminothiocarbonyl, dialkylaminothiocarbonyl, where the alkyl groups in these radicals contain 1 to 6 carbon atoms and the abovementioned alkenyl or alkynyl groups in these radicals contain 2 to 8 carbon atoms;
is C(=NOR^{d})-Γ₁-R^{d'}, where R^{d} is hydrogen or C₁-C₆-alkyl, Γ is oxygen, sulfur or NR^{d} and 1 is 0 or 1;
and/or one to three of the following radicals: cycloalkyl, cycloalkoxy, heterocyclyl, heterocyclyloxy, where the cyclic systems contain 3 to 10 ring members; phenyl, phenoxy, phenylthio, phenyl-C₁-C₆-alkoxy, phenyl-C₁-C₆-alkyl, hetaryl, hetaryloxy, hetarylthio, where the hetaryl radicals 5 or 6 ring members and where the cyclic systems may be partially or fully halogenated or may be substituted by one to three groups R^{b};
A is =N-OR⁴, =CH-OR⁴ =CH-SR⁴ or =CH-R⁵, where
R⁴ is C₁-C₄-alkyl or C₁-C₄-haloalkyl,
R⁵ is halogen or a group R⁴ and
W is C₁-C₃-alkylene which is unsubstituted or substituted by one or two groups R^{e}, where
R^{e} is halogen, cyano, C₁-C₄-alkyl, C₁-C₄-haloalkyl, C₂-C₄-alkenyl, C₂-C₄-haloalkenyl, C₂-C₄-alkynyl or C₂-C₄-haloalkynyl, C₁-C₄-alkoxy, C₁-C₄-haloalkoxy, C₂-C₄-alkenoxy, C₂-C₄-haloalkenyloxy, C₂-C₄-Alkynyloxy or C₂-C₄-haloalkynyloxy, C₁-C₄-alkylcarbonyloxy.

2. An azadioxacycloalkene of the formula I', 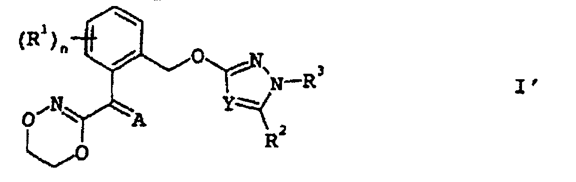 in which
R¹ is 6-Cl or 6-CH₃;
n is 0 or 1;
A is =N-OCH₃, =CH-OCH₃ or =CH-CH₃ and
Y, R² and R³ are as defined in claim 1.

3. A process for preparing compounds of the formula I as claimed in claim 1, which comprises reacting benzyl halides of the formula II, 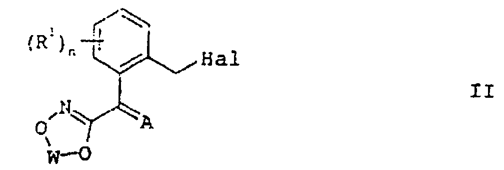 in which R¹, n, A and W are as defined in claim 1 and Hal is a halogen atom with compounds of the formula III, 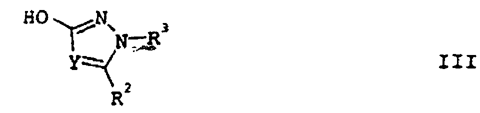 in which Y, R² and R³ are as defined in claim 1 in the presence of a base.

4. A process for preparing compounds of the formula I as claimed in claim 1, which comprises reacting phenylacetic acid derivatives of the formula IVa, 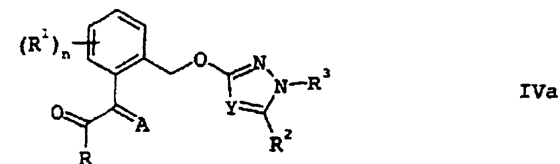 in which R¹, n, R², R³ and A are as defined in claim 1 and R is halogen, C₁-C₆-alkylcarbonyloxy, OH, C₁-C₄-alkoxy, phenoxy or azido, if appropriate in the presence of a base and/or a coupling reagent, with hydroxylamine or its acid addition salts to give hydroxamic acids of the formula IVb, 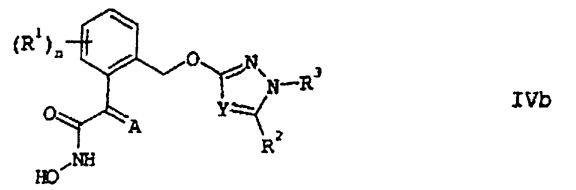 and reacting IVb with compounds of the formula IVa [sic],
L¹-W-L² Va
in which W is as defined in claim 1 and L¹ and L² are each a nucleophilically replaceable group, if appropriate in the presence of a base or of a dehydrating agent.

5. An intermediate of formula IVb as set forth in claim 4.

6. A composition suitable for controlling animal pests or harmful fungi, comprising a solid or liquid carrier and a compound of the formula I as claimed in claim 1.

7. The use of the compounds I as claimed in claim 1 for preparing a composition which is suitable for controlling animal pests or harmful fungi.

8. A method for controlling harmful fungi, which comprises treating the fungi or the materials, plants, the soil or the seed to be protected against fungal attack with an effective amount of a compound of the formula I as claimed in claim 1.

9. A method for controlling animal pests, which comprises treating the animal pests or the materials, plants, the soil or the seed to be protected against them with an effective amount of a compound of the formula I as claimed in claim 1.

## Revendications

1. Azadioxacyclo-alcènes de formule I 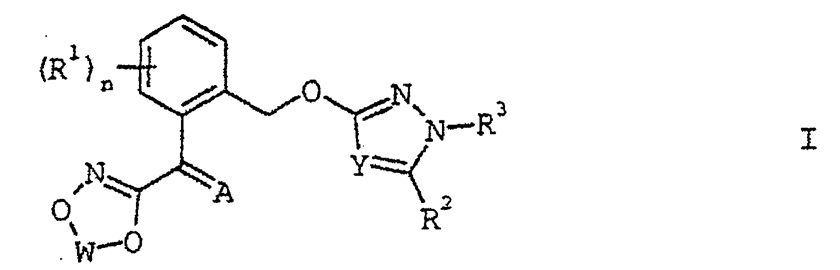 dans laquelle les substituants et l'indice présentent la signification suivante :
Y représente N ou CR^{a}, où
R^{a} représente un hydrogène, un halogène ou un alkyle en C₁-C₄ ;
n vaut 0, 1, 2, 3 ou 4, les substituants R¹ pouvant être différents lorsque n est supérieur à 1 ;
R¹ représente un nitro, un cyano, un halogène, un alkyle en C₁-C₆ ou
au cas où n est supérieur à 1, en outre un pont relié à deux atomes cycliques voisins, renfermant de trois à quatre chaînons choisis parmi le groupe constitué de : 3 ou 4 atomes de carbone, 2 à 3 atomes de carbone et 1 ou 2 atomes d'azote, d'oxygène et/ou de soufre, ce pont pouvant former, conjointement avec le noyau auquel il est lié, un radical partiellement insaturé ou aromatique et les atomes de carbone du pont pouvant en outre être partiellement ou totalement substitués par des atomes d'halogène ou des groupes méthyle ;
R² représente un hydrogène, un nitro, un cyano, un halogène, un alkyle en C₁-C₄, un halogénoalkyle en C₁-C₄, un alcoxy en C₁-C₄, un alkylthio en C₁-C₄, ou un C₁-C₄-alcoxycarbonyle ;
R³ représente un alkyle en C₁-C₆ qui est non substitué ou partiellement ou totalement halogéné, ou peut porter un à trois groupes R^{b},
Rb représente un halogène, un cyano, un nitro, un hydroxy, un alkyle en C₁-C₆, un halogénoalkyle en C₁-C₆, un C₁-C₆-alkylcarbonyle, un cycloalkyle en C₃-C₆, un alcoxy en C₁-C₆, un halogénoalcoxy en C₁-C₆, un C₁-C₆-alcoxycarbonyle, un alkylthio en C₁-C₆, un alkylamino en C₁-C₆, un di-C₁-C₆-alkylamino, un alcényle en C₂-C₆, un alcényloxy en C₂-C₆, un alcynyloxy en C₃-C₆ et un alkylènedioxy en C₁-C₄, qui peut être halogéné,
un cycloalkyle en C₃-C₆, un phényle, un naphtyle, un hétéroaryle à 5 chaînons, renfermant un à quatre atomes d'azote ou un à trois atomes d'azote et un atome de soufre ou d'oxygène, ou un hétéroaryle à 6 chaînons renfermant un à quatre atomes d'azote, les systèmes cycliques pouvant être partiellement ou totalement halogénés ou pouvant porter un à trois groupes R^{c} :
R^{c} représente un halogène, un cyano, un nitro, un hydroxy, un mercapto, un amino, un carboxy, un aminocarbonyle, un aminothiocarbonyle, un alkyle, un halogénoalkyle, un alcényle, un alcényloxy, un alcynyloxy, un alcoxy, un halogénoalcoxy, un alkylthio, un alkylamino, un dialkylamino, un formyle, un alkylcarbonyle, un alkylsulfonyle, un alkylsulfoxyle, un alcoxycarbonyle, un alkylcarbonyloxy, un alkylaminocarbonyle, un dialkylaminocarbonyle, un alkylaminothiocarbonyle, un dialkylaminothiocarbonyle, les groupes alkyle dans ces radicaux renfermant de 1 à 6 atomes de carbone et les groupes alcényle ou alcynyle mentionnés dans ces radicaux renfermant de 2 à 8 atomes de carbone ;
C(=NOR^{d})-Γ₁-R^{d'}, où R^{d} représente un hydrogène ou un alkyle en C₁-C₆, Γ représente un oxygène, un soufre ou NR^{d} et 1 vaut 0 ou 1 ;
et/ou un à trois des radicaux suivants :
un cycloalkyle, un cycloalcoxy, un hétérocyclyle, un hétérocyclyloxy, les systèmes cycliques renfermant de 3 à 10 chaînons cycliques ; un phényle, un phénoxy, un phénylthio, un phényl-C₁-C₆-alcoxy, un phényl-C₁-C₆-alkyle, un hétaryle, un hétaryloxy, un hétarylthio, les radicaux hétaryle renfermant 5 ou 6 chaînons cycliques, les systèmes cycliques pouvant être partiellement ou totalement halogénés et être substitués par un à trois groupes R^{b} ;
A représente =N-OR⁴, =CH-OR⁴, =CH-SR⁴ ou =CH-R⁵, où
R⁴ représente un alkyle en C₁-C₄ ou un halogénoalkyle en C₁-C₄,
R⁵ représente un halogène ou un groupe R⁴ et
W représente un alkylène en C₁-C₃ qui est non substitué ou substitué par un ou deux groupes R^{e}, où
R^{e} représente un halogène, un cyano, un alkyle en C₁-C₄, un halogénoalkyle en C₁-C₄, un alcényle en C₂-C₄, un halogénoalcényle en C₂-C₄, un alcynyle en C₂-C₄, ou un halogénoalcynyle en C₂-C₄, un alcoxy en C₁-C₄, un halogénoalcoxy en C₁-C₄, un alcénoxy en C₂-C₄, un halogénoalcénoxy en C₂-C₄, un alcynoxy en C₂-C₄, un halogénoalcynoxy en C₂-C₄ ou un C₁-C₄-alkylcarbonyloxy.

2. Azadioxacyclo-alcènes de formule I', 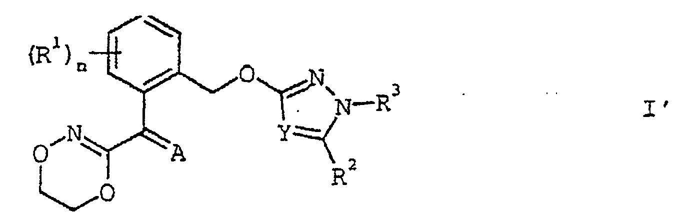 dans laquelle
R¹ représente 6-Cl ou 6-CH₃ ;
n vaut 0 ou 1 ;
A représente =N-OCH₃, =CH-OCH₃ ou =CH-CH₃ et
Y, R² et R³ présentent la signification indiquée dans la revendication 1.

3. Procédé de préparation des composés de formule I selon la revendication 1, **caractérisé en ce que** des halogénures de benzyle de formule II 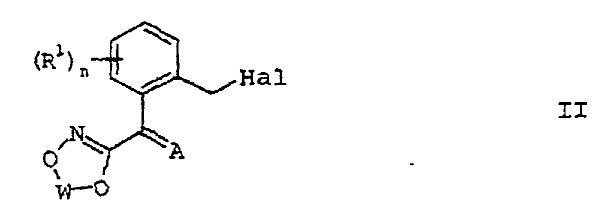 dans laquelle R¹, n, A et W présentent la signification indiquée dans la revendication 1 et Hal représente un atome d'halogène, sont mis à réagir avec des composés de formule III 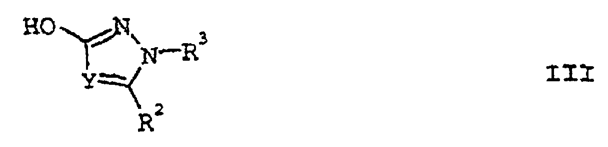 dans laquelle Y, R² et R³ présentent la signification indiquée dans la revendication 1, en présence d'une base.

4. Procédé de préparation des composés de formule I selon la revendication 1, **caractérisé en ce que** des dérivés de l'acide phénylacétique de formule IVa 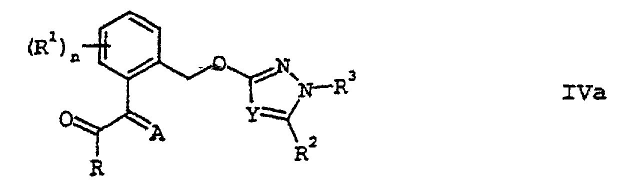 dans laquelle R¹, n, R², R³ et A présentent la signification indiquée dans la revendication 1 et R représente un halogène, un C₁-C₆-alkylcarbonyloxy, OH, un alcoxy en C₁-C₄, un phénoxy ou un azido, sont mis à réagir, éventuellement en présence d'une base et/ou d'un réactif de couplage, avec l'hydroxylamine ou ses sels d'addition à un acide pour donner lieu à des acides hydroxamiques de formule IVb, 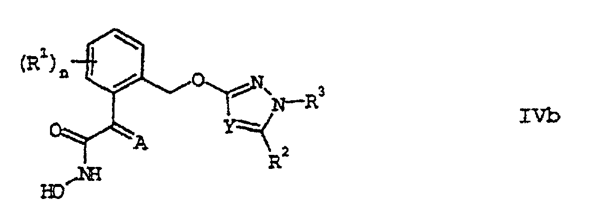 et les IVb sont mis à réagir avec des composés de formule IVa,
L¹-W-L² Va
dans laquelle W présente la signification mentionnée dans la revendication 1 et L¹ et L² représentent chacun un groupe remplaçable de façon nucléophile, éventuellement en présence d'une base ou d'un agent déshydratant.

5. Intermédiaire de formule IVb selon la revendication 4.

6. Composition appropriée pour la lutte des parasites animaux ou des champignons nuisibles, comprenant un support solide ou liquide et un composé de formule générale I selon la revendication 1.

7. Utilisation des composés I selon la revendication 1 pour la préparation d'une composition appropriée pour la lutte contre des parasites animaux ou des champignons nuisibles.

8. Procédé de lutte contre des champignons nuisibles, **caractérisé en ce que** les champignons ou les matériaux, les plantes, les sols ou les semences à protéger vis-à-vis d'une infection fongique sont traités par une quantité efficace d'un composé de formule générale I selon la revendication 1.

9. Procédé de lutte contre des parasites animaux, **caractérisé en ce que** les parasites animaux ou les matériaux, les plantes, les sols ou les semences à protéger vis-à-vis de ceux-ci sont traités par une quantité efficace d'un composé de formule générale I selon la revendication 1.
